(19) Europäisches Patentamt
European Patent Office
Office européen des brevets

(11) Numéro de publication : **0 030 511**
**B1**

(12) **FASCICULE DE BREVET EUROPÉEN**

(45) Date de publication du fascicule du brevet : **29.02.84**

(51) Int. Cl.[3] : **C 07 D307/86**

(21) Numéro de dépôt : **80420130.9**

(22) Date de dépôt : **01.12.80**

(54) **Procédé de préparation de dérivés de benzofuranne.**

(30) Priorité : **07.12.79 FR 7930645**

(43) Date de publication de la demande : **17.06.81 Bulletin 81/24**

(45) Mention de la délivrance du brevet : **29.02.84 Bulletin 84/09**

(84) Etats contractants désignés : **AT BE CH DE FR GB IT LI LU NL SE**

(56) Documents cités :
**FR-A- 1 548 441**
**CHEMICAL ABSTRACTS, vol. 91, no. 1 02-07-1979, page 479, résumé 5106c Columbus, Ohio, US**
**CHEMICAL ABSTRACTS, vol. 90, no. 3 15-01-1979, page 599, réf. 22471q Columbus, Ohio, US A. ALBEROLA et al.: "Rearrangement of aryl ethers with organoaluminum compounds"**
**Le dossier contient des informations techniques présentées postérieurement au dépôt de la demande et *ne figurant pas dans le présent fascicule*.**

(73) Titulaire : **RHONE-POULENC AGROCHIMIE**
**14-20, rue Pierre Baizet**
**F-69009 Lyon (FR)**

(72) Inventeur : **Michelet, Daniel**
**Les Erables No 21 24, Chemin de Montribloud**
**F-69160 Tassin La Demi Lune (FR)**
Inventeur : **Veracini, Serge**
**36, avenue de Ménival**
**F-69005 Lyon (FR)**

(74) Mandataire : **Chaumette, Michel et al**
**RHONE-POULENC AGROCHIMIE BP 9163-09**
**F-69263 Lyon Cedex 1 (FR)**

**EP 0 030 511 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de préparation de dérivés de benzofuranne

L'invention concerne un procédé de préparation du dihydro-2,3 diméthyl-2,2 hydroxy-7 benzofuranne.

Ce composé a pour formule :

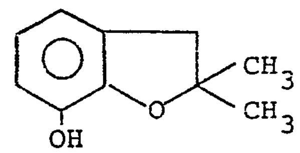

Le dihydro-2,3 diméthyl-2,2 hydroxy-7 benzofuranne, désigné dans ce qui suit par DDHB, est un composé en soi connu, utilisable pour la préparation du méthylcarbamate de dihydro-2,3 diméthyl-2,2 benzofurannyl-7, insecticide polyvalent connu sous le nom de carbofuran.

Il est connu d'après le brevet américain 3 474 171 et le brevet anglais 999 128 que le DDHB peut être obtenu par chauffage de l'orthométhallyloxyphénol, ce qui entraîne un réarrangement de ce composé par transposition du radical méthallyle avec formation de dérivés de la pyrocatéchine, puis cyclisation de certains des dérivés ainsi formés (notamment de l'orthométallylpyrocatéchine ou métallyl-3 dihydroxy-1,2 benzène) pour donner le DDHB.

Selon ces brevets, cette transformation s'effectue par chauffage dans la masse de l'orthométhallyloxyphénol à température élevée sans utilisation de catalyseur, avec, d'une façon générale, de faibles rendements en DDHB (48,2 % de la théorie selon le brevet anglais 999 128).

L'orthométallyloxyphénol est le composé de formule :

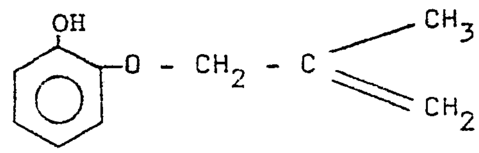

L'orthométhallylpyrocatéchine est le composé de formule :

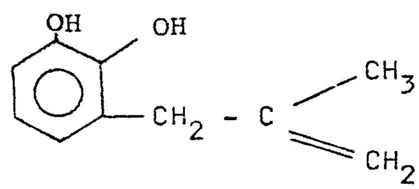

Il est également connu, d'après Stroh et al. Neuere Methoden der Präparativen Org. Chem. II p-231-246 et en particulier p. 245, que le chauffage du tert-butyl-phényléther en présence de phénolates d'aluminium entraîne une scission de cet éther avec libération d'isobutène et fixation de cet isobutène, sur le phénol, pour donner l'ortho-tert-butylphénol.

Le procédé décrit par Stroh correspond à une alkylation de phénols, par une oléfine, s'orientant spécifiquement vers une alkylation sur le noyau, en position ortho par rapport à l'hydroxyle. Il implique, de plus, qu'une oléfine puisse se former par scission de l'alkyl-phényl éther de départ.

L'orthométhallyloxyphénol présente la particularité de posséder à la fois un groupe phénol et une insaturation de type oléfinique. L'application de conditions opératoires telles que décrites par Stroh au chauffage de l'orthométhallyloxyphénol aurait donc normalement dû conduire à une réaction d'alkylation en ortho par rapport au groupe hydroxyle, pouvant s'écrire :

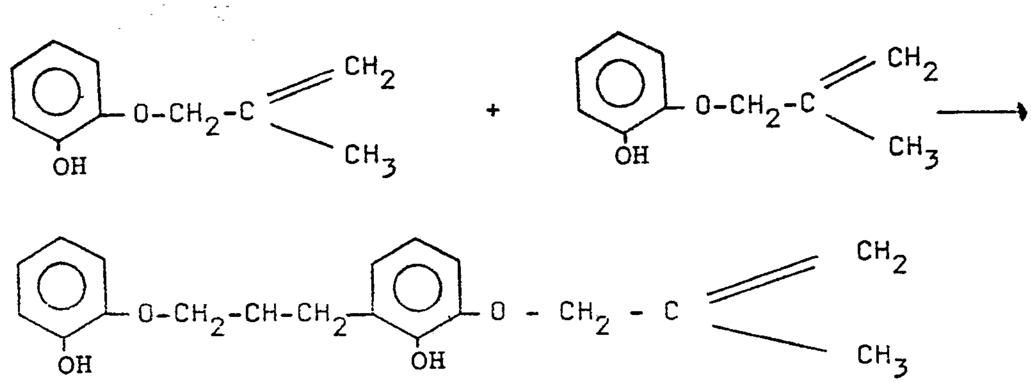

Il est évident que la réaction que se propose l'invention a une nature entièrement différente de celle décrite par Stroh, puisque, selon l'invention, il y a fixation d'une chaîne hydrocarbonée en méta et non pas en ortho.

Il est connu enfin, d'après le brevet français 1 548 441 de préparer des dihydro-2,3 diméthyl-2,2 alcoxy-7 benzofurannes à partir d'alkoxy-1 méthallyloxy-2 benzènes par réarrangement de ces composés en alkoxy-2 méthallyl-6 phénols, puis cyclisation de ces composés.

Le procédé décrit par ce brevet est différent de celui revendiqué par notre demande du fait que le produit final aussi bien que le produit de départ et celui intermédiairement formé sont des dérivés « alkoxy » différents de ceux mis en œuvre selon notre demande. Par ailleurs, ce brevet ne décrit ni ne suggère d'aucune façon l'utilisation, comme catalyseurs, des dérivés d'aluminium proposés par la présente demande de brevet.

Un but de la présente invention est de fournir un procédé amélioré de préparation du DDHB, notamment à partir d'orthométhallylpyrocatéchine.

Un autre but de l'invention est de préparer le DDHB à partir de l'orthométhallyloxyphénol avec un rendement amélioré.

Un autre but de l'invention est de permettre de préparer essentiellement en une seule étape le DDHB à partir d'orthométhallyloxyphénol, sans avoir à isoler le ou les composés formés intermédiairement.

Il a maintenant été trouvé que ces buts pouvaient être atteints grâce au nouveau procédé qui fait l'objet de la présente invention.

Ce procédé est un procédé de préparation du DDHB, par chauffage d'orthométhallylpyrocatéchine ; il est caractérisé en ce que l'orthométhallylpyrocatéchine est chauffée en présence, comme catalyseur, d'un dérivé d'aluminium.

Dans ces conditions, on observe que, de façon inattendue, le DDHB est obtenu avec un rendement élevé, à des températures relativement basses (par exemple 100 à 150 °C), alors qu'en l'absence de dérivé d'aluminium, le chauffage de l'orthométhallylpyrocatéchine, à ces mêmes températures, ne conduit qu'à des rendements, en DDHB, insignifiants, voire même à l'absence de toute formation de DDHB.

L'orthométhallylpyrocatéchine peut être préparée par isomérisation thermique de l'orthométhallyloxyphénol, de type transposition de Claisen, au moyen d'un chauffage à une température généralement très élevée, (de l'ordre de 200 °C environ) avantageusement en présence d'un solvant organique inerte.

Selon un mode de réalisation préférentiel du procédé de l'invention, le réarrangement de l'orthométhallyloxyphénol en orthométhallylpyrocatéchine s'effectue en chauffant l'orthométhallyloxyphénol en présence d'un dérivé d'aluminium. On observe alors que la présence de ce dérivé d'aluminium pendant le chauffage de l'orthométhallyloxyphénol favorise, de façon inattendue, le réarrangement de ce composé. Il est donc particulièrement avantageux de chauffer l'orthométhallyloxyphénol en présence d'un dérivé d'aluminium et de poursuivre ce chauffage en présence du même dérivé d'aluminium jusqu'à ce qu'il y ait formation du DDHB. Dans ces conditions, on observe que les deux transformations successives s'enchaînent immédiatement et que l'orthométhallylpyrocatéchine se transforme au fur et à mesure de sa formation, en DDHB.

On obtient ainsi un procédé permettant de produire le DDHB en une seule étape, à partir de l'orthométhallyloxyphénol en chauffant celui-ci en présence d'un dérivé d'aluminium.

Ce procédé, qui fait partie de l'invention, concerne donc la préparation du DDHB par chauffage de l'orthométhallyloxyphénol ; il est caractérisé en ce que l'orthométhallyloxyphénol est chauffé en présence d'un dérivé d'aluminium. Il constitue le mode de réalisation préféré de l'invention.

Selon un deuxième mode de réalisation du procédé de l'invention, l'orthométhallyloxyphénol est d'abord chauffé en l'absence de dérivé de l'aluminium, à température élevée, et il se produit alors, une transformation partielle ou totale du produit de départ en dérivés de la pyrocatéchine (orthométhallylpyrocatéchine notamment), puis le chauffage du milieu réactionnel contenant ces produits intermédiairement formés est poursuivi en présence d'un dérivé d'aluminium, que l'on ajoute alors au milieu réactionnel.

On obtient ainsi également un procédé permettant de préparer le DDHB en une seule étape à partir de l'orthométhallyloxyphénol, sans avoir à isoler les produits intermédiairement formés. Ce procédé qui fait également partie de l'invention concerne la préparation du DDHB par chauffage d'orthométhallyloxyphénol ; il est caractérisé en ce que l'on chauffe tout d'abord l'orthométhallyloxyphénol, à température très élevée, en l'absence de dérivé d'aluminium, jusqu'à transformation de la majeure partie de l'orthométhallyloxyphénol, puis que l'on poursuit ultérieurement le chauffage en présence d'un dérivé d'aluminium.

Les dérivés d'aluminium utilisés selon le procédé de l'invention sont des composés de formule :

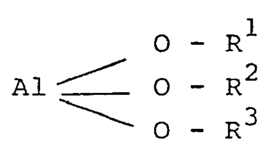

dans laquelle $R_1$, $R_2$ et $R_3$, identiques ou différents, sont choisis parmi les radicaux suivants :

— alcoyle éventuellement halogéné, contenant de 1 à 20 atomes de carbone,

— cycloalcoyle ou cycloalcoyle-alcoyle contenant de 3 à 12 atomes de carbone (par exemple cyclohéxylméthyle),

— aralcoyle contenant de 7 à 10 atomes de carbone (par exemple benzyle),

— aryle contenant de 6 à 12 atomes de carbone (par exemple phényle, naphthyle et diphényle) éventuellement substitué par 1 à 3 substituants, identiques ou différents, choisis parmi les atomes d'halogènes, les radicaux hydroxy, alcoyle ou alcoyloxy contenant de 1 à 4 atomes de carbone, alcényle ou alcényloxy (linéaires ou ramifiés) contenant de 2 à 6 atomes de carbone, et phényle,

— benzofuryle éventuellement substitué par 1 à 4 radicaux, identiques ou différents, choisis parmi les radicaux alcoyle contenant de 1 à 4 atomes de carbone et alcényle contenant de 2 à 4 atomes de carbone (par exemple l'un ou plusieurs des radicaux $R_1$, $R_2$ et $R_3$ peuvent représenter le radical dihydro-2,3 diméthyl-2,2 benzofuryle-7).

Comme dérivés d'aluminium, on utilise de préférence soit des alcoolates d'aluminium, notamment des alcoylates inférieurs d'aluminium, c'est-à-dire dont chacune des parties alcoyle contient au plus six atomes de carbone, tels que, par exemple, l'isopropylate d'aluminium, soit des phénolates d'aluminium tels que, par exemple, le dérivé de substitution par l'aluminium du phénol ordinaire, le crésolate d'aluminium et l'orthométhallyloxyphénolate d'aluminium, éventuellement préparés in situ. Au sens de la présente demande, l'expression phénolate d'aluminium désigne d'une façon générale les dérivés de substitution par l'aluminium des différents phénols et ne doit donc pas être restreinte au seul dérivé du phénol ordinaire. Parmi ces dérivés d'aluminium, on utilise, de préférence l'isopropylate d'aluminium.

Les alcoolates et phénolates d'aluminium utilisables dans le procédé de l'invention peuvent être préparés selon des méthodes en soi connues, par exemple, par action de l'aluminium sur l'alcool ou le phénol approprié, en milieu anhydre. Ainsi, le phénolate de formule $(C_6H_5O)_3$ Al peut être préparé in situ par action de poudre d'aluminium sur le phénol. Les phénolates d'aluminium peuvent également être préparés par action d'un alcoylate ou phénolate d'aluminium sur le phénol approprié.

La qualité de dérivés d'aluminium à utiliser doit être suffisante pour permettre à la transformation de s'effectuer de façon satisfaisante. Pour cela, on utilise généralement au moins 0,000 5 mole de dérivé d'aluminium par mole de produit de départ à transformer, c'est-à-dire respectivement par mole d'orthométhallylpyrocatéchine lorsque ce composé est utilisé comme produit de départ, ou par mole d'orthométhallyloxyphénol lorsque ce composé est utilisé comme produit de départ, selon les variantes du procédé de l'invention. La limite supérieure de la quantité de catalyseur à utiliser n'est pas critique. De préférence toutefois, on utilise de 0,001 à 0,3 mole de dérivé d'aluminium par mole de produit de départ à transformer.

La température à laquelle est effectué le chauffage de l'orthométhallylpyrocatéchine/ou de l'orthométhallyloxyphénol, en présence du dérivé d'aluminium, est avantageusement comprise entre 80° et 250 °C ; elle est de préférence comprise entre 100 et 200 °C.

La transformation de l'orthométhallyloxyphénol ou de l'orthométhallylpyrocatéchine, en DDHB, selon le procédé de l'invention s'effectue de façon satisfaisante en l'absence de tout solvant.

Elle peut également être effectuée avantageusement en présence d'un solvant organique de préférence choisi parmi les hydrocarbures aromatiques tels que le toluène, les o-, met p-xylènes ; les hydrocarbures aliphatiques tels que l'octane ou le décane ; les hydrocarbures aliphatiques ou aromatiques chlorés tels que le dichloro-1,2 éthane ou le trichloro-1,1,2 éthane ; les hydrocarbures cycloaliphatiques tels que le cyclohéxane ; les éthers aliphatiques ou aromatiques tels que l'anisole ; les cétones telles que la méthylisobutylcétone ; les alcools tels que les alcanols inférieurs comme par exemple l'isopropanol, les phénols et plus particulièrement le phénol proprement dit.

La transformation selon l'invention s'effectue avantageusement sous pression atmosphérique ordinaire. Elle peut toutefois être effectuée sous une pression différente de la pression atmosphérique ordinaire. Ainsi, lorsqu'on désire utiliser un solvant dont la température d'ébullition, sous pression atmosphérique normale, est inférieure à la température à laquelle on désire opérer, la transformation selon l'invention peut être effectuée en opérant en autoclave, sous une pression supérieure à la pression atmosphérique.

La durée nécessaire pour effectuer la transformation selon l'invention dépend de la température utilisée, c'est-à-dire que cette durée est d'autant plus courte que la température est plus élevée. Cette durée est généralement comprise entre 15 minutes et vingt heures.

En fin de réaction, le DDHB obtenu est séparé par tout moyen connu en soi tel que par exemple par distillation. Toutefois, pour certaines utilisations, il peut ne pas être nécessaire d'isoler le DDHB et il suffit alors de le laisser dans le milieu réactionnel qui est lui-même mis en œuvre de la manière souhaitée. Le dérivé d'aluminium peut être éliminé en lavant la masse réactionnelle par une solution aqueuse d'un acide fort.

L'orthométhallyloxyphénol est un composé en soi connu dont la préparation a été décrite dans la demande de brevet français N° 2 255 279.

Les exemples suivants donnés à titre non limitatif illustrent l'invention et montrent comment elle peut être mise en œuvre.

Pour plus de commodité, dans les exemples suivants, la notation « TT (ME) » signifiera « taux de transformation de l'orthométhallyloxyphénol » et la notation « RT (DDHB) » signifiera « rendement en DDHB par rapport à l'orthométhallyloxyphénol transformé ».

## Exemple 1

L'appareillage utilisé est constitué d'un ballon de 20 ml muni d'un réfrigérant ascendant et d'un barreau magnétique.

On y charge successivement :

- 0,020 7 g d'isopropylate d'aluminium ($1,0.10^{-4}$ mole),
- 1,701 2 g d'o-méthallyloxyphénol ($1,037.10^{-2}$ mole).

Le mélange est chauffé à 130 °C pendant 12 heures, sous agitation, puis refroidi à température ambiante. On ajoute alors 20 ml d'une solution aqueuse 2N d'acide chlorhydrique, 20 ml d'acétate d'éthyle, et on agite vigoureusement l'ensemble dans une ampoule à décanter. Après séparation des deux phases, on lave la phase organique par 20 ml d'eau distillée qu'on réunit ensuite à la phase aqueuse ; cette phase aqueuse est à son tour lavée par 20 ml d'acétate d'éthyle qu'on réunit à la première phase organique. L'ensemble des phases organiques fournit 1,208 g de DDHB et contient de plus 0,195 g d'orthométhallyloxyphénol non transformé soit :

- TT (ME) = 88,5 %
- RT (DDHB) = 80,2 %

## Essai comparatif

En opérant selon les conditions de température et traitement décrites dans l'exemple 1, à partir de 1,043 g d'orthométhallyloxyphénol, sans utiliser de dérivé d'aluminium, on obtient un mélange contenant :

- 0,189 g d'orthométhallyloxyphénol non transformé,
- 0,012 6 g de DDHB,
- 0,520 g d'orthométhallylpyrocatéchine,
- 0,188 g de paraméthallylpyrocatéchine, soit :

- TT (ME) = 81,9 %
- RT (DDHB) = 1,5 %

(De cet essai, on observera qu'à 130 °C, en l'absence de catalyseur, la cyclisation de l'orthométhallylpyrocatéchine en DDHB ne s'effectue que dans une proportion très faible et que le TT (ME) est inférieur à celui obtenu avec catalyseur.)

## Exemple 2

On opère selon la même méthode qu'à l'exemple 1, en utilisant le même appareillage, à partir de :

- 0,043 6 g d'isopropylate d'aluminium ($2,1.10^{-4}$ mole),
- 1,716 4 g d'o-méthallyloxyphénol ($1,046.10^{-2}$ mole).

Le mélange est chauffé à 140 °C pendant 6 heures sous agitation.

On obtient ainsi 1,080 g de DDHB et il reste 0,343 g d'orthométhallyloxyphénol non transformé, soit :

- TT (ME) = 80,0 %
- RT (DDHB) = 78,6 %

## Exemple 3

On opère selon la même méthode qu'à l'exemple 1, en utilisant le même appareillage, à partir de :

- 0,025 7 g d'isopropylate d'aluminium ($1,2.10^{-4}$ mole),
- 2,278 9 g d'o-méthallyloxyphénol ($1,4.10^{-2}$ mole).

Le mélange est chauffé à 150 °C pendant 1 heure sous agitation :

- TT (ME) = 75,1 %
- RT (DDHB) = 75,4 %

## Exemple 4

On opère selon la même méthode qu'à l'exemple 1, en utilisant le même appareillage, à partir de :

— 0,239 g d'isopropylate d'aluminium ($11,5.10^{-4}$ mole),
— 1,754 8 g d'orthométhallyloxyphénol ($1,07.10^{-2}$ mole).

Le mélange est chauffé à 150 °C pendant 15 minutes sous agitation :

— TT (ME) = 96,4 %
— RT (DDHB) = 71,7 %

Exemple 5

Dans l'appareillage décrit à l'exemple 1, on charge successivement :

— 0,266 g d'isopropylate d'aluminium ($13,0.10^{-4}$ mole),
— 2,156 g d'o-méthallyloxyphénol ($1,31.10^{-2}$ mole),
— 5 ml de p. xylène.

Le mélange est chauffé à 140 °C pendant 2 h 30 sous agitation, puis refroidi à température ambiante. On ajoute alors 50 ml d'une solution aqueuse 2N d'acide chlorhydrique, 50 ml de chlorure de méthylène, et on agite l'ensemble dans une ampoule à décanter. Après filtration sur büchner pour éliminer les insolubles et séparation des deux phases liquides, on lave la phase aqueuse avec 2 fois 25 ml de chlorure de méthylène que l'on réunit ensuite à la phase organique.

L'ensemble des phases organiques fournit 1,666 g de DDHB et contient 0,072 g d'orthométhallyloxyphénol non transformé soit :

— TT (ME) = 96,6 %
— RT (DDHB) = 79,9 %

Exemple 6

On opère selon la même méthode qu'à l'exemple 5, en utilisant le même appareillage à partir de :

— 0,244 g d'isopropylate d'aluminium ($12.10^{-4}$ mole),
— 2,165 8 g d'o-méthallyloxyphénol ($1,32.10^{-2}$ mole),
— 5 ml de p. xylène.

Le mélange est chauffé à 120 °C pendant 3 heures 30 sous agitation. On obtient ainsi :

— 1,456 g de DDHB et il reste :
— 0,298 g d'orthométhallyloxyphénol non transformé soit :

— TT (ME) = 86,2 %
— RT (DDHB) = 78,0 %

Exemple 7

On opère selon la même méthode qu'à l'exemple 5, en utilisant le même appareillage, à partir de :

— 0,025 2 g d'isopropylate d'aluminium ($1,2.10^{-4}$ mole),
— 2,438 4 g d'orthométhallyloxyphénol ($1,5.10^{-2}$ mole),
— 6 ml de xylène.

Le mélange est chauffé à 140 °C pendant 16 h 30, sous agitation :

— TT (ME) = 98,5 %
— RT (DDHB) = 74,0 %

Exemple 8

On opère selon la même méthode qu'à l'exemple 5, en utilisant le même appareillage, à partir de :

— 0,132 g d'isopropylate d'aluminium ($6,4.10^{-4}$ mole),
— 2,002 g d'orthométhallyloxyphénol ($1,2.10^{-2}$ mole),
— 5 ml d'anisole.

Le mélange est chauffé à 130 °C pendant 6 heures, sous agitation :

— TT (ME) = 86,9 %
— RT (DDHB) = 74,8 %

Exemple 9

On opère selon la même méthode qu'à l'exemple 5, en utilisant le même appareillage, à partir de :

— 0,822 g d'isopropylate d'aluminium ($40.10^{-4}$ mole),
— 2,119 g d'orthométhallyloxyphénol ($1,3.10^{-2}$ mole),
— 5 ml de n-décane.

Le mélange est chauffé à 100 °C pendant 1 heure, sous agitation. On obtient un mélange contenant :

— 1,286 g de DDHB,
— 0,177 g d'orthométhallylpyrocatéchine ($0,11.10^{-2}$ mole), mais ne contenant plus d'orthométhallyloxyphénol :

— TT (ME) = 100,0 %
— RT (DDHB) = 60,7 %

Exemple 10

Dans un autoclave en acier inoxydable de 25 $cm^3$, on charge successivement :

— 0,237 g d'isopropylate d''aluminium ($11,6.10^{-4}$ mole),
— 2,047 3 g d'o-méthallyloxyphénol ($1,24.10^{-2}$ mole),
— 6 ml d'alcool isopropylique.

L'autoclave est chauffé à 150 °C pendant 3 heures dans un bain constitué d'un alliage de Wood, puis refroidi à température ambiante. Le mélange réactionnel est alors partiellement concentré, puis repris entre 50 ml d'une solution aqueuse d'acide chlorhydrique 2N et 50 ml de chlorure de méthylène. La suite du traitement est identique à celle décrite dans l'exemple N° 5.

On obtient ainsi :

— 1,564 g de DDHB et il reste :
— 0,036 g d'orthométhallyloxyphénol non transformé, soit :

— TT (ME) = 98,2 %
— RT (DDHB) = 77,8 %

Exemple 11

On opère selon la même méthode qu'à l'exemple 10, dans un autoclave dans lequel on charge :

— 0,263 g d'isopropylate d'aluminium ($12,9.10^{-4}$ mole),
— 2,008 7 g d'orthométhallyloxyphénol ($1,22.10^{-2}$ mole),
— 6 ml de n-décane.

Le mélange est chauffé à 150 °C pendant 3 heures, puis traité comme indiqué à l'exemple 10 :

— TT (ME) = 98,6 %
— RT (DDHB) = 73,5 %

Exemple 12

On opère selon la même méthode qu'à l'exemple 10, dans un autoclave dans lequel on charge :

— 0,263 g d'isopropylate d'aluminium ($12,9.10^{-4}$ mole),
— 2,284 g d'orthométhallyloxyphénol ($1,4.10^{-2}$ mole),
— 6 ml de p. xylène.

Le mélange est chauffé à 200 °C pendant 30 minutes, puis traité comme indiqué à l'exemple 10 :

— TT (ME) = 99,6 %
— RT (DDHB) = 71,0 %

Exemple 13

(illustre la préparation du catalyseur *in situ*) :

Dans l'appareillage, décrit à l'exemple N° 1, on charge sucessivement :

— 0,039 g de poudre d'aluminium ($14,3.10^{-4}$ mole) préalablement dépassivé par traitement à l'acide chlorhydrique aqueux,

— 2,186 g d'o-méthallyloxyphénol ($1,33.10^{-2}$ mole),

— 10 ml de p. xylène.

Le mélange est chauffé à 140 °C. On ajoute alors 1 mg environ de chlorure mercurique et un dégagement gazeux se produit instantanément. On maintient le mélange réactionnel à 140 °C pendant 1 heure 30 sous agitation, puis on le refroidit à température ambiante. Le traitement est identique à celui décrit dans l'exemple N° 5.

On obtient ainsi :

— 1,093 g de DDHB et il reste :

— 0,768 g d'orthométhallyloxyphénol non transformé soit :

— TT (ME) = 64,9 %

— RT (DDHB) = 77,1 %

Exemple 14

Dans l'appareillage, décrit à l'exemple 1, on charge :

— 0,030 5 g ($1,0.10^{-4}$ mole) de $(C_6H_5O)_3Al$, préparé dans une opération séparée par action de l'isopropylate d'aluminium sur le phénol,

— 1,601 7 g d'o-méthallyloxyphénol ($9,77.10^{-3}$ mole).

On chauffe pendant 15 heures à 130 °C. On traite comme indiqué dans l'exemple 1, et on obtient ainsi :

— 0,338 g d'o-méthallyloxyphénol non transformé,

— 0,975 g de DDHB soit :

— TT (ME) = 78,9 %

— RT (DDHB) = 75,6 %

Exemple 15

Dans un autoclave en titane, on charge 12,0 g d'orthométhallyloxyphénol titrant 96,4 % et contenant de plus de 1,17 % de DDHB et 108 g de n-octane. On chauffe pendant 1 h 30 à 200 °C et on refroidit. La masse réactionnelle est ensuite concentrée jusqu'à élimination complète du n-octane. L'analyse du produit brut obtenu (11,93 g) montre alors que l'orthométhallyloxyphénol a disparu du milieu réactionnel et qu'il s'est formé essentiellement des méthallylpyrocatéchines (principalement de l'orthométhallylpyrocatéchine).

Dans l'appareillage décrit à l'exemple 1, on charge successivement :

— 0,013 g d'isopropylate d'aluminium ($0,6.10^{-4}$ mole),

— 1,387 1 g du produit brut obtenu précédemment.

Le mélange est chauffé à 130 °C pendant 5 h 30 mn sous agitation puis refroidi à température ambiante et le traitement est alors poursuivi comme indiqué dans l'exemple 1.

On obtient ainsi une solution dans l'acétate d'éthyle contenant 0,960 g de DDHB. Cette solution ne contient pas d'orthométhallyloxyphénol ni d'orthométhallylpyrocatéchine non transformée soit :

— RT (DDHB) = 70,1 %

**Revendications**

1. Procédé de préparation de dihydro-2,3 diméthyl-2,2 hydroxy-7 benzofuranne par chauffage d'orthométhallylpyrocatéchine, caractérisé en ce que l'orthométhallylpyrocatéchine est chauffée en présence d'un dérivé de l'aluminium, répondant à la formule :

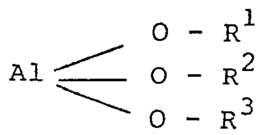

dans laquelle $R_1$, $R_2$ et $R_3$, identiques ou différents, représentent chacun un radical :

— alcoyle éventuellement halogéné contenant de 1 à 20 atomes de carbone,

— cycloalcoyle ou cycloalcoyl-alcoyle contenant de 3 à 12 atomes de carbone,

— aralcoyle contenant de 7 à 10 atomes de carbone,

— aryle contenant de 6 à 12 atomes de carbone, éventuellement substitué par 1 à 3 substituants, identiques ou différents, choisis parmi les atomes d'halogènes les radicaux hydroxy, alcoyles contenant de 1 à 4 atomes de carbone, alcoyloxy contenant de 1 à 4 atomes de carbone, alcényles contenant de 2 à 6 atomes de carbone, alcényloxy, contenant de 2 à 6 atomes de carbone et phényle,

— benzofuryle, éventuellement substitué par 1 à 4 radicaux identiques ou différents, choisis parmi les radicaux alcoyles contenant de 1 à 4 atomes de carbone et alcényle contenant de 2 à 4 atomes de carbone.

2. Procédé selon la revendication 1, caractérisé en ce que l'orthométhallylpyrocatéchine est obtenue par chauffage de l'orthométhallyloxyphénol.

3. Procédé selon la revendication 2, caractérisé en ce que l'orthométhallylpyrocatéchine est obtenue par chauffage de l'orthométhallyloxyphénol en présence du dérivé d'aluminium.

4. Procédé selon la revendication 3, caractérisé en ce que la transformation de l'orthométhallyloxyphénol en dihydro-2,3 diméthyl-2,2 hydroxy-7 benzofuranne, s'effectue en une seule opération par chauffage en présence du dérivé d'aluminium.

5. Procédé selon l'une des revendications 1 à 4, caractérisé en ce que le chauffage, en présence du dérivé d'aluminium, est effectué à une température comprise entre 80 et 250 °C.

6. Procédé selon la revendication 5, caractérisé en ce qu'on utilise au moins 0,000 5 mole de dérivé d'aluminium par mole de produit de départ à transformer.

7. Procédé selon la revendication 6, caractérisé en ce que le dérivé d'aluminium est un alcoolate d'aluminium ou un phénolate d'aluminium.

8. Procédé selon la revendication 7, caractérisé en ce que le dérivé d'aluminium est choisi parmi les alcoylates inférieurs d'aluminium, le phénolate d'aluminium, le crésolate d'aluminium et l'orthométhallyloxyphénolate d'aluminium.

9. Procédé selon la revendication 8, caractérisé en ce que l'on utilise de 0,001 à 0,3 mole de dérivé d'aluminium par mole de produit de départ à transformer.

10. Procédé selon la revendication 9, de préparation de dihydro-2,3 diméthyl-2,2 hydroxy-7 benzofuranne à partir d'orthométhallyloxyphénol, caractérisé en ce que l'orthométhallyloxyphénol, est chauffé en présence du dérivé d'aluminium, à une température comprise entre 100° et 200 °C.

11. Procédé selon la revendication 10, caractérisé en ce que le dérivé d'aluminium est l'isopropylate d'aluminium.

12. Procédé selon l'une des revendications 1 à 11, caractérisé en ce que le chauffage du produit de départ, orthométhallylpyrocatéchine ou orthométhallyloxyphénol, est effectué en présence d'un solvant organique.

## Claims

1. A process for the preparation of 2.3-dihydro-2.2-dimethyl-7-hydroxybenzofuran by heating orthomethallylpyrocatechol, characterized in that the ortho-methallylpyrocatechol is heated in the presence of an aluminium derivative corresponding to the formula :

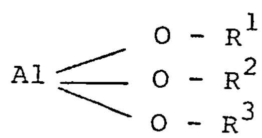

in which $R_1$, $R_2$ and $R_3$, which are identical or different, each represent

— an optionally halogen-substituted alkyl radical containing from 1 to 20 carbon atoms,

— a cycloalkyl or cycloalkyl-alkyl radical containing from 3 to 12 carbon atoms,

— an aralkyl radical containing from 7 to 10 carbon atoms,

— an aryl radical containing from 6 to 12 carbon atoms, which is optionally substituted by 1 to 3 identical or different substituents chosen from amongst halogen atoms, hydroxy radicals, alkyl radicals containing from 1 to 4 carbon atoms, alkoxy radicals containing from 1 to 4 carbon atoms, alkenyl radicals containing from 2 to 6 carbon atoms, alkenyloxy radicals containing from 2 to 6 carbon atoms, and phenyl radicals, or

— the benzofuryl radical which is optionally substituted by 1 to 4 identical or different radicals chosen from amongst alkyl radicals containing from 1 to 4 carbon atoms and alkenyl radicals containing from 2 to 4 carbon atoms.

2. A process according to claim 1, characterized in that the ortho-methallylpyrocatechol is obtained by heating ortho-methallyloxyphenol.

3. A process according to claim 2, characterized in that the ortho-methallylpyrocatechol is obtained by heating ortho-methallyloxyphenol in the presence of the aluminium derivative.

4. A process according to claim 3, characterized in that the conversion of ortho-methallyloxyphenol to give 2.3-dihydro-2.2-dimethyl-7-hydroxybenzofuran is carried out in a single operation by heating in the presence of the aluminium derivative.

5. A process according to one of claims 1 to 4, characterized in that the heating in the presence of the aluminium derivative is carried out at a temperature between 80 and 250 °C.

6. A process according to claim 5, characterized in that at least 0.000 5 mol of aluminium derivative is used per mol of starting material to be converted.

7. A process according to claim 6, characterized in that the aluminium derivative is an aluminium alcoholate or an aluminium phenolate.

8. A process according to claim 7, characterized in that the aluminium derivative is chosen from amongst aluminium lower alkylates, aluminium phenolate, aluminium cresolate and aluminium ortho-methallyloxyphenolate.

9. A process according to claim 8, characterized in that from 0.001 to 0.3 mol of aluminium derivative is used per mol of starting material to be converted.

10. A process according to claim 9 for the preparation of 2.3-dihydro-2.2-dimethyl-7-hydroxybenzofuran from ortho-methallyloxyphenol, characterized in that orthomethallyloxyphenol is heated in the presence of the aluminium derivative, at a temperature between 100° and 200 °C.

11. A process according to claim 10, characterized in that the aluminium derivative is aluminium isopropylate.

12. A process according to one of claims 1 to 11, characterized in that the heating of the starting material, i. e. ortho-methallylpyrocatechol or orthomethallyloxyphenol, is carried out in the presence of an organic solvent.

**Ansprüche**

1. Verfahren zur Herstellung von 2,3-Dihydro-2,2-dimethyl-7-hydroxybenzofuran durch Erhitzen von o-Methallylbrenzcatechin, dadurch gekennzeichnet, daß das o-Methallylbrenzkatechin in Gegenwart einer Aluminiumverbindung der Formel :

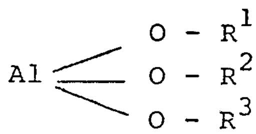

erhitzt wird, in der $R^1$, $R^2$ und $R^3$ gleich oder verschieden sind und jeweils eine der folgenden Bedeutungen haben :

— gegebenenfalls halogenierte Alkylgruppe mit 1 bis 20 Kohlenstoffatomen,

— Cycloalkyl- oder Cycloalkyl-alkylgruppe mit 3 bis 12 Kohlenstoffatomen,

— Aralkylgruppe mit 7 bis 10 Kohlenstoffatomen,

— Arylgruppe mit 6 bis 12 Kohlenstoffatomen, gegebenenfalls substituiert durch 1 bis 3 Substituenten, die gleich oder verschieden sind und ausgewählt aus der Gruppe Halogenatome, Hydroxygruppe, Alkylgruppen mit 1 bis 4 Kohlenstoffatomen, Alkoxygruppen mit 1 bis 4 Kohlenstoffatomen, Alkenylgruppen mit 2 bis 6 Kohlenstoffatomen, Alkenyloxygruppen mit 2 bis 6 Kohlenstoffatomen, und Phenylgruppe,

— Benzofurylgruppe, gegebenenfalls substituiert durch 1 bis 4 gleiche oder verschiedene Substituenten, ausgewählt aus Alkylgruppen mit 1 bis 4 Kohlenstoffatomen und Alkenylgruppen mit 2 bis 4 Kohlenstoffatomen.

2. Verfahren nach Anspruch 1, dadurch gekennzeichnet, dass das o-Methallylbrenzcatechin durch Erhitzen von o-Methallyloxyphenol erhalten wird.

3. Verfahren nach Anspruch 2, durch gekennzeichnet, dass das o-Methallylbrenzcatechin durch Erhitzen von o-Methallyloxyphenol in Gegenwart einer Aluminiumverbindung erhalten wird.

4. Verfahren nach Anspruch 3, dadurch gekennzeichnet, dass die Umwandlung von o-Methallyloxyphenol in 2,3-Dihydro-2,2-dimethyl-7-hydroxybenzofuran in einem einzigen Arbeitsgang mittels Erhitzen in Gegenwart einer Aluminiumverbindung vorgenommen wird.

5. Verfahren nach einem der Ansprüche 1 bis 4, dadurch gekennzeichnet, dass in Gegenwart der Aluminiumverbindung auf eine Temperatur von 80 bis 250 °C erhitzt wird.

6. Verfahren nach Anspruch 5, dadurch gekennzeichnet, dass mindestens 0,000 5 mol Aluminiumverbindung je mol Ausgangsverbindung, welche umgewandelt werden soll, eingesetzt werde.

7. Verfahren nach Anspruch 6, dadurch gekennzeichnet, dass die Aluminiumverbindung ein Aluminiumalkoholat oder Aluminiumphenolat ist.

8. Verfahren nach Anspruch 7, dadurch gekennzeichnet, dass die Aluminiumverbindung ausgewählt wird aus der Gruppe der niederen Aluminiumalkoholate, Aluminiumphenolat, Aluminiumkresolat und Aluminium-o-methallyloxyphenolat.

9. Verfahren nach Anspruch 8, dadurch gekennzeichnet, dass 0,001 bis 0,3 mol Aluminiumverbindung je mol Ausgangsverbindung, die umgewandelt werden soll, eingesetzt werden.

10. Verfahren nach Anspruch 9 zur Herstellung von 2,3-Dihydro-2,2-dimethyl-7-hydroxybenzofuran, ausgehend von o-Methallyloxyphenol, dadurch gekennzeichnet, dass das o-Methallyloxyphenol in Gegenwart der Aluminiumverbindung auf eine Temperatur im Bereich von 100 bis 200 °C erhitzt wird.

11. Verfahren nach Anspruch 10, dadurch gekennzeichnet, dass die Aluminiumverbindung Aluminiumisopropylat ist.

12. Verfahren nach einem der Ansprüche 1 bis 10, dadurch gekennzeichnet, dass die Ausgangsverbindung o-Methallylbrenzcatechin oder o-Methallyloxyphenol in Gegenwart eines organischen Lösungsmittels erhitzt wird.